# EUROPÄISCHE PATENTANMELDUNG

(11) **EP 4 009 098 A2**
(43) Veröffentlichungstag der Anmeldung: **08.06.2022**
(21) Anmeldenummer: 21207396.9
(22) Anmeldetag: 10.11.2021
(51) Int. Cl.: G02C 7/08, A61F 2/16, G02C 7/04

(54) **SELBST AKKOMMODIERENDE LINSE SOWIE VERFAHREN ZU DEREN STEUERUNG**

(30) Priorität: 11.11.2020 DE 102020129721
(71) Anmelder: Binder, Helmut, 10707 Berlin (DE)
(72) Erfinder: Binder, Helmut, 10707 Berlin (DE)
(74) Vertreter: Friderichs, Gunther

(57) **Zusammenfassung**

Die Offenbarung bezieht sich auf eine selbst akkommodierende Linse, welche insbesondere als Kontaktlinse ausgebildet ist. Auf der Vorderseite des Linsenkörpers sind eine Vielzahl von Aktuatoren sternförmig angeordnet. Der Winkel zu der benachbarten Linse wird durch Detektion eines gerichteten Funksignals bestimmt.

## Beschreibung

### Gebiet der Erfindung

Die Erfindung betrifft eine selbst akkommodierende Linse. Die Linse ist insbesondere als Kontaktlinse ausgebildet. Weiter betrifft die Erfindung ein Verfahren zur Steuerung einer selbst akkommodierenden Linse.

### Hintergrund der Erfindung

Selbst akkommodierende Linsen, also Linsen, welche die Brennweite ändern können, sind bekannt.

So beschreibt die Offenlegungsschrift WO 2010/133317 A1 (Dr. Helmut Binder) eine selbst akkommodierende Linse mit einem Positionssensor.

Gemäß der Lehre dieser Offenlegungsschrift wird der Winkel der optischen Achsen zwischen zwei Linsen gemessen und anhand des gemessenen Winkels die Krümmung der Linse verändert, um so die Brennweite an die Entfernung anzupassen.

Gegenüber beispielsweise Kontaktlinsen, welche Ringsegmente mit unterschiedlicher Brennweite aufweisen, hat eine selbst akkommodierende Kontaktlinse den Vorteil, dass das gesamte, auf der Netzhaut abgebildete Bild von einer einzigen Linse mit im Wesentlichen einheitlicher Brennweite stammt. Weiter gibt es keine Kontrastverluste und auch keine störende Streuungseffekte.

Dies macht die Gewöhnung des Benutzers an die Kontaktlinse einfacher und darüber hinaus bleibt die Lichtstärke des Auges vollständig erhalten.

Es ist wünschenswert, dass selbst akkommodierende Kontaktlinsen insbesondere bei Alterssichtigkeit oder in Verbindung mit einer nicht akkommodierenden Intraokularlinse nach einer Katarakt-Operation Verwendung finden.

Die technische Umsetzung eines solchen Systems ist allerdings schwierig. Insbesondere schwierig ist die Integration der benötigten Komponenten für die Winkelmessung sowie der Änderung der Krümmung der Linse.

Das Dokument US 2014/0243971 A1 zeigt eine selbst akkommodierende Linse, wobei der Winkel zwischen zwei Linsen mittels eines Sensors messbar ist.

### Aufgabe der Erfindung

Der Erfindung liegt demgegenüber die Aufgabe zugrunde, die Integration eines mechanischen Systems zur Änderung des Krümmungsradius einer Linse und damit der Brechzahl und/oder die Messung des Winkels zwischen zwei benachbarten Linsen zu verbessern.

### Zusammenfassung der Erfindung

Die Aufgabe der Erfindung wird bereits durch eine selbst akkommodierende Linse sowie durch ein Verfahren zur Steuerung einer selbst akkommodierenden Linse nach einem der unabhängigen Ansprüche gelöst.

Bevorzugte Ausführungsformen und Weiterbildungen der Erfindung sind dem Gegenstand der abhängigen Ansprüche, der Beschreibung sowie den Zeichnungen zu entnehmen.

Gemäß einem ersten Aspekt betrifft die Erfindung eine selbst akkommodierende Linse.

Die Linse ist insbesondere als Kontaktlinse ausgebildet, also als Linse, welche auf die Hornhaut aufgesetzt wird und sich dort festsaugt.

Prinzipiell kann die Erfindung aber auch für eine Intraokularlinse, also eine Linse, welche als Implantat zum Ersatz der menschlichen Linse ausgebildet ist, verwendet werden.

Die Linse umfasst einen Linsenkörper, welcher zumindest teilweise aus einem transparenten elastischen Material ausgebildet ist.

Insbesondere kann die Linse aus einem Silikon, insbesondere aus einem Silikon-Hydrogel, ausgebildet sein.

Das Material der Linse lässt sich verformen, um so die Brennweite in Abhängigkeit vom Abstand zu dem Objekt, auf welches der Benutzer der Linse blickt, zu ändern.

Die Linse umfasst einen Linsenkörper mit einer Vorderseite und einer Rückseite. Die Rückseite ist die dem Benutzer zugewandte, also die proximale Seite, wohingegen die Vorderseite, die dem Benutzer abgewandte Seite, also die distale Seite, darstellt, welche bei einer Kontaktlinse insbesondere als Außenseite ausgebildet ist.

Gemäß der Erfindung ist auf der Vorderseite der Linse zumindest ein Aktuator zur Veränderung der Krümmung der Linse angeordnet.

Es hat sich herausgestellt, dass bereits durch die Veränderung der Krümmung der Linse von deren Vorderseite ausgehend eine Veränderung der Brechzahl auf einfache Weise möglich ist.

Weiter wird bei einem elastischen Körper die Krümmung der Vorderseite durch den auf der Vorderseite vorhandenen Aktuators stärker verändert als auf der Rückseite.

Die Rückseite, die der Krümmung des Auges angepasst sein kann, verändert sich beim Ändern der Brennweite weniger oder gar nicht.

Hierdurch ist Änderung der Brennweite einer selbst akkommodierenden Kontaktlinse für den Träger nicht oder zumindest nur wenig spürbar.

Unter der Anordnung des zumindest einen Aktuators auf der Vorderseite wird verstanden, dass der Aktuator derart angeordnet ist, dass dieser unmittelbar die Krümmung auf der Vorderseite, insbesondere der Außenseite einer Kontaktlinse verändert.

Der Aktuator kann dabei die Außenseite der Linse bei bestimmungsgemäßem Gebrauch bilden.

Weiter kann der oder die Aktuatoren mit einer transparenten Schicht, insbesondere aus demselben Material wie der restliche Linsenkörper versehen sein.

Vorzugsweise umfasst die Linse eine Mehrzahl von Aktuatoren.

Die Linse kann insbesondere 2 bis 20, vorzugsweise 4 bis 16, ganz besonders bevorzugt 6 bis 12 Aktuatoren umfassen.

Die Aktuatoren sind vorzugsweise streifenförmig ausgebildet und ändern ihre Krümmung in Abhängigkeit von einem Steuersignal.

Insbesondere erstrecken sich die Aktuatoren sternförmig über die Vorderseite der Linse.

Über auf der Vorderseite angebrachte mikromechanische Bauelemente kann die Krümmung der Linse auf sehr einfache Weise verändert werden.

Die Aktuatoren können sich insbesondere sternförmig über die Vorderseite der Linse erstrecken.

Bei geeignet schmalen Aktuatoren ist es dabei auch möglich, dass sich die Aktuatoren im optischen Zentrum der Linse berühren und/oder überlappen.

So können insbesondere streifenförmige Aktuatoren sternförmig von der Mitte, also vom optischen Zentrum ausgehend, verteilt sein.

Bei einer anderen Ausführungsform der Erfindung sind die Aktuatoren um einen ausgesparten mittigen Bereich, insbesondere um einen kreisförmigen mittigen Bereich, herum angeordnet.

Diese Ausführungsform der Erfindung hat zwar den Nachteil, dass die Krümmung der Linse und damit die Änderung der Brechzahl mit geringerer Effizienz verändert werden kann. Gleichzeitig ist aber das optische Zentrum frei von Aktuatoren.

Gemäß einer bevorzugten Ausführungsform der Erfindung ist die akkommodierende Linse derart ausgebildet, dass die Krümmung schrittweise verstellbar ist, insbesondere in 2 bis 5 Schritten, vorzugsweise in genau 3 Schritten.

Es hat sich gezeigt, dass eine stufenlose Akkommodation nicht notwendig ist. Vielmehr reichen bereits wenige, fest definierte, unterschiedliche Brennweiten aus, um über den gesamten Sichtbereich eine hinreichende Akkommodation vorzunehmen.

Die schrittweise Veränderung der Brennweite ermöglicht eine steuerungstechnisch einfachere Ansteuerung der Aktuatoren.

Dies gilt insbesondere in Verbindung mit einem gerichteten Funksignal, auf welches sich ein weiterer Aspekt der Erfindung bezieht.

So muss nämlich auch messtechnisch lediglich das Über- bzw. Unterschreiten von Schwellwerten detektiert werden, nicht aber fortlaufend der Winkel der optischen Achsen berechnet werden.

Die erfindungsgemäße Linse lässt sich so energiesparender betreiben.

Dies gilt insbesondere für eine Ausführungsform der Erfindung, bei welcher die Linse derart ausgebildet ist, dass diese in einer Fernsicht-Stufe Aktuatoren umfasst, die in ihrer Ruheposition sind.

Erst, wenn der Benutzer seine Augen nicht mehr auf Fernsicht stellt, müssen die Aktuatoren von einer Steuerungseinheit angesteuert werden und nur dann wird hierfür entsprechende Energie benötigt.

Der Aktuator kann insbesondere als mikroelektromechanisches System (MEMS) ausgebildet sein.

Derartige Aktuatoren können insbesondere nach dem elektrostatischen Prinzip arbeiten.

So kann der zumindest eine Aktuator insbesondere ein biegsames Substrat umfassen, welches insbesondere auch Teil des Linsenkörpers sein kann.

Über sich gegenüberliegende Elektroden, zwischen denen im Substrat ein Hohlraum angeordnet ist, kann ein derartiger Aktuator auf einfache Weise seine Krümmung verändern.

Auf der Vorderseite der Linse angeordnet, wird so die Krümmung der Linse mit verändert, so dass die Brennweite verändert werden kann.

Als Eingangsgröße für den Aktuator wird vorzugsweise ein Winkel zu einer anderen Linse verwendet.

Es versteht sich, dass der Winkel mit dem Abstand im Mittelpunkt der Linse korreliert.

Der Winkel kann insbesondere über einen Sensor bestimmt werden.

Weiter kann die Linse einen Energiespeicher umfassen, insbesondere einen Energiespeicher, der in Dünnschichttechnik auf das Substrat, insbesondere auf den Linsenkörper, aufgebracht ist.

Der Energiespeicher kann insbesondere auch als mehrschichtiger Kondensator ausgebildet sein.

Insbesondere kann ein sog. Superkondensator (engl. supercap) als Energiespeicher verwendet werden.

Dieser kann insbesondere als Doppelschichtkondensator mit Kohlenstoffelektroden, als Pseudokondensator mit Elektroden aus Metalloxiden oder aus leitfähigen Polymeren oder als sog. Hybridkondensator mit asymmetrischen Elektroden ausgebildet sein.

Gemäß einer bevorzugten Ausführungsform der Erfindung lässt sich die Linse induktiv laden.

Die hierfür erforderliche Induktionsspule kann sich insbesondere konzentrisch über die Vorderseite der Linse erstrecken.

Gemäß einem weiteren Aspekt betrifft die Erfindung eine selbst akkommodierende Linse, insbesondere eine Kontaktlinse und welche insbesondere die vorstehend beschriebenen Merkmale aufweisen kann.

Die Linse umfasst einen Linsenkörper, dessen Krümmung in Abhängigkeit zu dem Winkel zu einer benachbarten Linse veränderbar ist.

Die Krümmung kann insbesondere, wie vorstehend beschrieben, über zumindest einen Aktuator verändert werden.

Gemäß der Erfindung umfasst die selbst akkommodierende Linse zur Messung des Winkels zur benachbarten Linse eine Antenne, welche ein gerichtetes Signal abstrahlt.

Das gerichtete Signal, welches insbesondere auch als Signalkeule definiert werden kann, wird von einer gegenüberliegenden Kontaktlinse erfasst.

Anhand der Signalstärke kann der Winkel erfasst werden.

Bei einer stufen- bzw. schrittweisen Verstellung der Krümmung der Linse über einen Aktuator können vordefinierte Schwellwerte verwendet werden, bei deren Unter- bzw. Überschreitung ein Steuersignal am Aktuator verändert wird.

Vorzugsweise ist steuerungstechnisch um den Schwellwert herum eine Hystereseschleife vorgesehen, um zu verhindern, dass der Aktuator um den Schwellwert herum ständig mit einem anderen Signal angesteuert wird.

Um die Signalstärke zu messen, kann dieselbe Antenne verwendet werden, welche gleichzeitig ein gerichtetes Funksignal in Richtung der anderen Linse abstrahlt.

Weiter kann gemäß einer weiteren Ausführungsform der Erfindung der Winkel dadurch bestimmt werden, dass mehrere voneinander beabstandete Antennen vorhanden sind, wobei bestimmt wird, auf welcher Antenne das Funksignal am stärksten ist.

So kann insbesondere eine schrittweise Ansteuerung des Aktuators vorgenommen werden.

Die Antenne ist gemäß einer bevorzugen Ausführungsform als Patch-Antenne ausgebildet.

Derartige Patch-Antennen umfassen eine vorzugsweise rechteckig ausgebildete Schicht auf einem Substrat.

Als Substrat kann insbesondere der Linsenkörper dienen.

Über die Verwendung eines Patch-Arrays, also einer regelmäßigen Anordnung mit einer Vielzahl von Antennen, kann die Aussendung eines gerichteten Funksignals verbessert werden.

Vorzugsweise sind hinter den jeweiligen Patches Antennenreflektoren vorgesehen.

Die Patch-Antenne kann insbesondere auch als gekrümmtes Patch-Array ausgebildet sein, welches in Dünnschichttechnik auf der Linse aufgebracht ist.

Die Erfindung betrifft des Weiteren ein Verfahren zur Steuerung der Brennweite einer selbst akkommodierenden Linse, insbesondere wie diese zuvor beschrieben wurde.

Gemäß der Erfindung wird die Signalstärke eines gerichteten Funksignals einer benachbarten Linse gemessen.

Auf Basis der Signalstärke des Funksignals wird mittels einer Steuereinrichtung ein Steuersignal für zumindest ein Aktuator, vorzugsweise eine Vielzahl von Aktuatoren, berechnet.

Der zumindest eine Aktuator bzw. die Aktuatoren wird/werden zur Veränderung der Krümmung der Linse mit dem Steuersignal angesteuert.

Die Steuereinrichtung kann insbesondere den Winkel der optischen Achse zweiter benachbarter Linsen berechnen und so den zumindest einen Aktuator auf Basis des Winkels ansteuern.

Wie vorstehend beschrieben, erfolgt die Steuerung vorzugsweise stufenweise, insbesondere zumindest 2 Stufen und/oder weniger als 10 Stufen, insbesondere in 2, 3, 4 oder 5 Stufen.

Wie bereits vorstehend ausgeführt, ist vorzugsweise eine Fernsichtstufe vorhanden, in welcher der zumindest eine Aktuator in einer Ruheposition ist.

Strom zur Ansteuerung der Aktuatoren wird daher nur benötigt, wenn sich das Sichtfeld in einen Nahbereich bewegt.

Diese Funktion bildet in ähnlicher Weise die Funktion des menschlichen Auges nach, bei welchem sich der Sehmuskel im Nahbereich anspannt.

Die Erfindung betrifft des Weiteren ein Set mit zwei selbst akkommodierenden Linsen, welches zumindest eine selbst akkommodierende Linse umfasst, wie sie vorstehend beschrieben wurde.

Vorzugsweise umfasst das Set zwei der vorstehend beschriebenen Linsen, insbesondere kann jede Linse sowohl über einen Sender als auch über einen Empfänger verfügen.

### Kurzbeschreibung der Zeichnungen

Der Gegenstand der Erfindung soll im Folgenden bezugnehmend auf schematisch dargestellte Ausführungsbeispiele anhand der Zeichnungen Fig. 1 bis Fig. 11 näher erläutert werden.
Fig. 1 und Fig. 2 zeigen jeweils in einer schematischen Draufsicht auf die Vorderseite verschiedene Ausführungsbeispiele einer selbst akkommodierenden Linse.
Fig. 3 ist eine Detailansicht der Antenne.
Fig. 4 zeigt ein Patch-Array.
Fig. 5 ist eine symbolische Darstellung der Antennen beim Senden und Empfangen.
Fig. 6 ist eine Simulation des Funksignals einer Antenne, welche als Patch-Array ausgebildet ist.
Fig. 7 zeigt schematisch einen für die Erfindung verwendeten elektrostatischen Aktuator.
Fig. 8 ist eine Illustration der Augen des Benutzers mit zwei erfindungsgemäßen selbst akkommodierenden Linsen.
Fig. 9 zeigt den Zusammenhang der Entfernung eines Objektes und der hierfür erforderlichen Dioptrie.
Fig. 10 ist ein Flussdiagramm eines Ausführungsbeispiels des erfindungsgemäßen Verfahrens zur Steuerung einer selbst akkommodierenden Linse.
Fig. 11 ist eine schematische Darstellung einer weiteren Ausführungsform einer selbst akkommodierenden Linse.

### Detaillierte Beschreibung der Zeichnungen

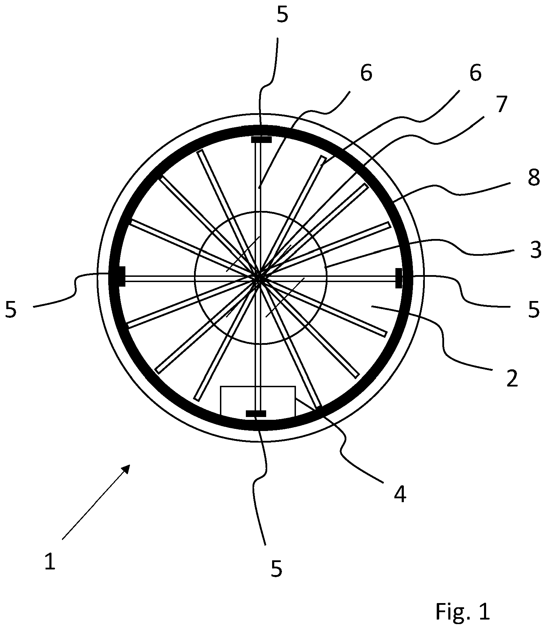

Fig. 1 zeigt in einer Draufsicht auf die Vorderseite schematisch darstellt eine erfindungsgemäße selbst akkommodierende Linse 1.

Die selbst akkommodierende Linse 1 umfasst einen Linsenkörper 2 aus einem transparenten Material, insbesondere einem Silikon.

Wenn die Linse 1 ins menschliche Auge eingesetzt wird, so erstreckt sich um einen Mittelpunkt 7, auf welchem die optische Achse liegt, ein Bereich 3, durch welchen bei maximaler Öffnung der Iris Licht in das Auge fällt.

Es versteht sich, dass insbesondere in diesem Bereich die Form des Linsenkörpers 2 geändert werden muss, um eine andere Brennweite zu erzielen.

Hierzu umfasst die Linse 1 eine Vielzahl von Aktuatoren 6, welche sich sternförmig nahezu über den gesamten Linsenkörper 2 erstrecken.

Die Aktuatoren 6 sind auf der Vorderseite des Linsenkörpers 2 angebracht.

Die Aktuatoren 6 sind streifenförmig und können die Krümmung der Vorderseite der Linse 1 verändern.

Um zu detektieren, auf welche Entfernung die Augen des Benutzers (nicht dargestellt) fokussiert sind, kann der Winkel zu einer benachbarten Linse 1 gemessen werden.

Hierzu kann die Linse eine Mehrzahl von Patch-Antennen 5 umfassen, welche insbesondere als Patch-Array ausgebildet sind.

Die Signalkeule zumindest einer Patch-Antenne 5 kann von einer benachbarten Linse erfasst und gemessen werden, wie im Folgenden noch ausführlicher dargestellt wird.

Zur Energieversorgung umfasst in diesem Ausführungsbeispiel der Linsenkörper 2 einen ringförmigen Dünnschicht-Akku 8, welcher sich im äußeren Bereich konzentrisch um die Aktuatoren 6 erstreckt.

Konzentrisch zum Dünnschicht-Aktuator können auch Leiterbahnen zur Ansteuerung der Aktuatoren 6 sowie eine Spule zum induktiven Aufladen des Akkus 8 angeordnet sein.

Eine Steuerungseinrichtung 4 mit den entsprechenden Prozessoren zur Signalauswertung und Ansteuerung der Aktuatoren kann auf der Vorderseite des Linsenkörpers 2 angeordnet sein oder in den Linsenkörper 2 eingelassen sein.

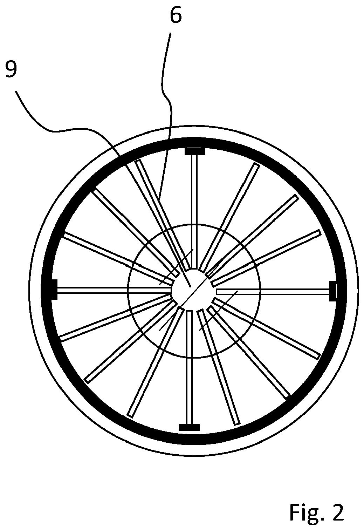

Fig. 2 zeigt ein alternatives Ausführungsbeispiel der Erfindung.

Bei diesem Ausführungsbeispiel der Erfindung erstrecken sich die Aktuatoren 6 nicht durch den Mittelpunkt der optischen Achse, sondern es ist ein freier Bereich 9 vorgesehen, um welchen sich die Aktuatoren 6 strahlenförmig erstrecken.

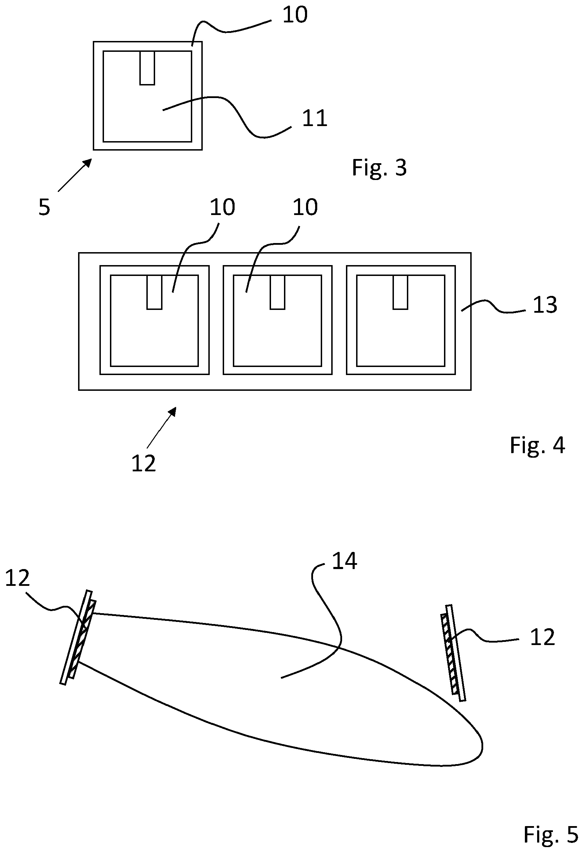

Fig. 3 ist eine schematische Ansicht einer Patch-Antenne 5.

Die Patch-Antenne umfasst eine rechteckig ausgebildete Antenne 10, welche auf einem Reflektor angeordnet ist.

Zum Erreichen einer guten Richtwirkung wird vorzugsweise ein Patch-Array 12 verwendet, welches eine Mehrzahl von Antennen 10 umfasst, die auf einem Substrat 13 angeordnet sind.

Wie schematisch in Fig. 5 dargestellt, kann über das Patch-Array 12 eine Signalkeule 14 ausgestrahlt werden, welche von einem gegenüberliegenden Patch-Array 12 erfasst wird.

Anhand der Signalstärke kann auf den Winkel zwischen den optischen Achsen der Linse geschlossen werden.

In diesem Ausführungsbeispiel liegt sich jeweils nur eine Antenne in Form eines Patch-Array gegenüber.

Bei einer anderen Ausführungsform (nicht dargestellt) sind mehrere voneinander beabstandete Antennen vorhanden und es wird detektiert, auf welche Antenne die Signalkeule 14 gerichtet ist.

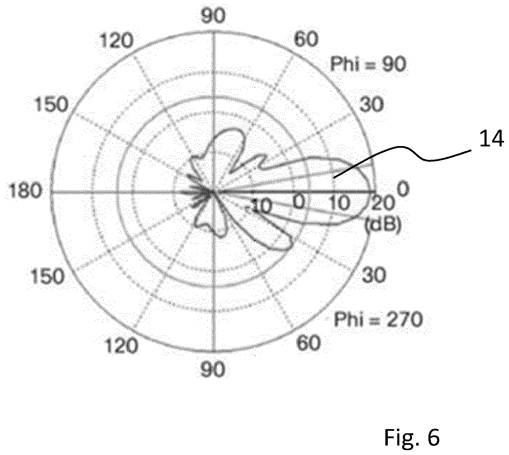

Fig. 6 zeigt die Simulation der Signalkeule 14 eines Patch-Arrays.

Zu erkennen ist, dass ein derartiges Patch-Array eine gute Richtwirkung hat. Insbesondere erstreckt sich ein Anteil mit hoher Signalstärke lediglich über einen Winkelbereich von etwa 30°.

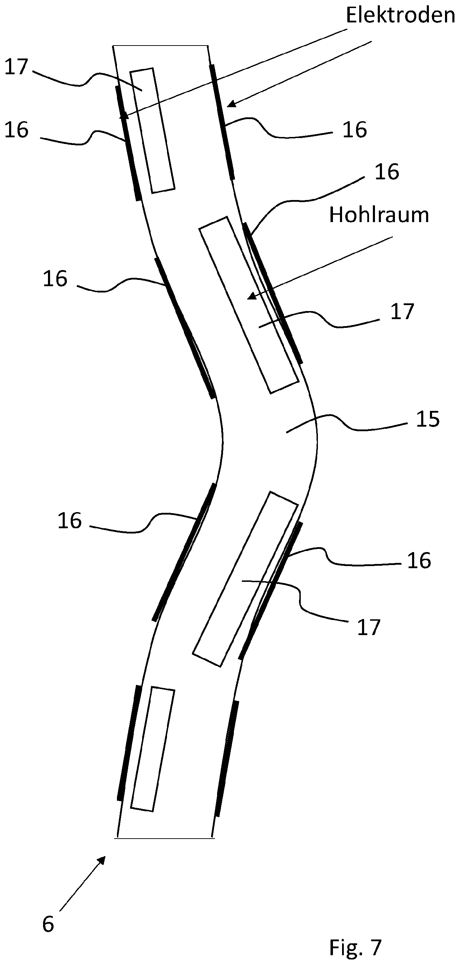

Fig. 7 zeigt in einer schematischen Seitenansicht das Grundprinzip eines Ausführungsbeispiels eines für die Erfindung verwendeten Aktuators.

Der Aktuator umfasst ein biegsames Substrat 15.

Dieses ist vorzugsweise streifenförmig ausgebildet und kann auch Teil des Linsenkörpers sein.

Auf Vorder- und Rückseite des Substrats 15 sind eine Vielzahl von gegenüberliegenden Elektrodenpaaren 16 angeordnet.

Zwischen den Elektrodenpaaren 16 befindet sich jeweils ein Hohlraum 17.

Der Hohlraum 17 erstreckt sich vorzugsweise streifenförmig und parallel entlang der Elektroden 16.

Über die Position des Hohlraums 17 und die Ansteuerung der Elektroden 16 kann auf elektrostatischer Basis die Krümmung des Substrats 15 verändert werden.

Gezeigt ist in diesem schematischen Ausführungsbeispiel der Ruhezustand, also der Zustand, wenn keine Spannung an den Elektroden 16 anliegt.

Das biegsame Substrat 15 folgt dabei der Grundkontur des Linsenkörpers.

Wird nun eine Spannung angelegt, so können die Hohlräume 17 quer zu ihrer Haupterstreckungsrichtung zusammengepresst oder auseinandergezogen werden, was dazu führt, dass sich die Krümmung des biegsamen Substrats 15 und damit die Krümmung an der Oberseite des Linsenkörpers ändert.

Eine Veränderung der Oberseite des Linsenkörpers zieht eine Konturveränderung hinsichtlich der Konvexität insgesamt nach sich und verändert so die Brennweite der Linse.

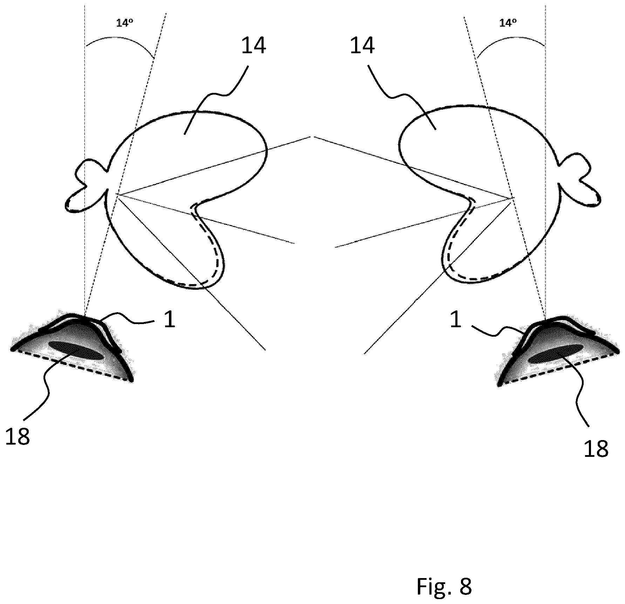

In Fig. 8 ist das Funktionsprinzip der Erfindung schematisch dargestellt.

Dargestellt sind die zwei sich gegenüberliegenden Augen 18 des Benutzers, welche hier in etwas übertriebener Darstellung auf den Nahbereich fokussiert sind.

Die Augen 18 stehen so in relativ großem Winkel nach innen.

Der Benutzer trägt zwei erfindungsgemäße Kontaktlinsen 1.

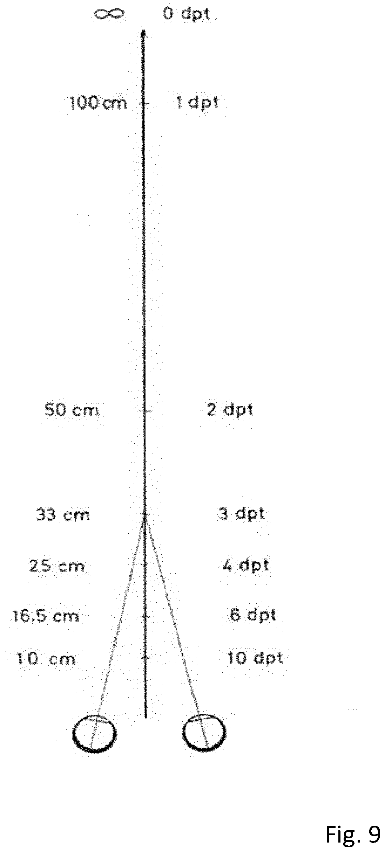

Zu sehen ist, dass die schematisch dargestellten Funkkeulen 14 je nach Winkel der Augen 18 in eine andere Richtung strahlen. Fig. 9 zeigt den Zusammenhang zwischen Nahsicht und Fernsicht und der hierfür erforderlichen Dioptrie.

Bei einem gesunden Normalsichtigen ist auch die Linse des menschlichen Auges auf Fernsicht auf eine Ruheposition eingestellt.

Kann sich die Linse nicht mehr verformen oder auch nach einer Katarakt-Operation, ist für das Nahsehfeld, welches ab etwa 1 m beginnt, eine Anpassung der Dioptrie notwendig.

Es versteht sich, dass die hier dargestellte Dioptrie bis 10 cm nicht notwendig ist.

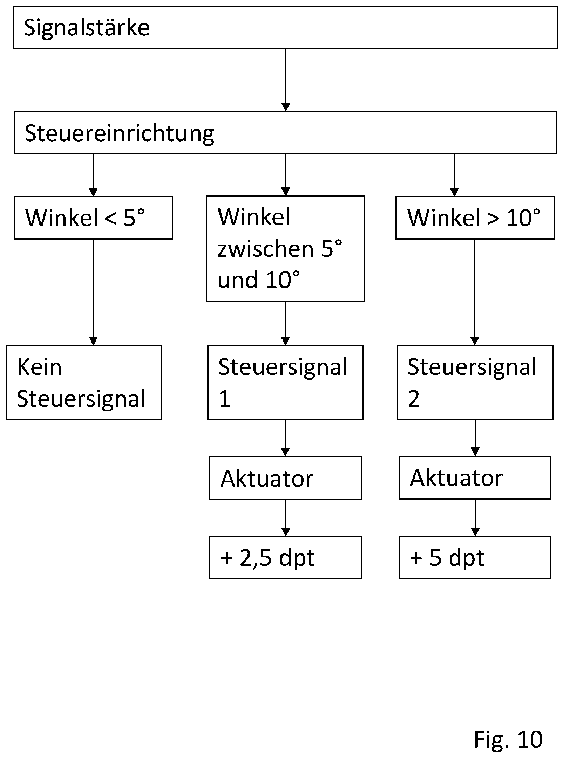

Ein Ausführungsbeispiel des erfindungsgemäßen Verfahrens zur Steuerung einer Linse, insbesondere einer Kontaktlinse, ist in Fig. 10 dargestellt.

Mittels einer Antenne, insbesondere mit einer Patch-Antenne, wird die Signalstärke der Funkkeule der benachbarten Linse gemessen.

Über eine Steuereinrichtung wird nunmehr bestimmt, in welchem Winkel die Augen relativ zur optischen Achse inkliniert sind.

Ein Winkel von unter 5° lässt darauf schließen, dass die Augen des Benutzers auf Fernsicht eingestellt sind, beispielsweise, weil der Benutzer ein Objekt in der Ferne fokussiert.

Die Aktuatoren verbleiben dabei in der Ruheposition und über die Steuereinrichtung wird kein Steuersignal an die Aktuatoren abgegeben.

Beim Wechsel ins Nahfeld können die Aktuatoren in zwei Stufen verändert werden.

Bei einem Winkel zwischen 5° und 10° wird über ein erstes Steuersignal durch Ansteuerung der Aktuatoren die Brennweite derart verändert, so dass die Dioptrie um 2,5 zunimmt.

Bei einem Winkel von 10° schaltet die Steuereinrichtung auf maximale Nahsicht und es wird ein zweites Steuersignal erzeugt, durch welches die Aktuatoren die Dioptrie um 5 erhöhen.

Bereits eine derartige Modulation in drei Stufen genügt, um ohne zusätzliche Sehhilfe sowohl Objekte in der Ferne scharf fokussieren zu können als auch um im Nahsichtfeldbereich Bildschirmarbeit verrichten zu können und normale Textgröße lesen zu können.

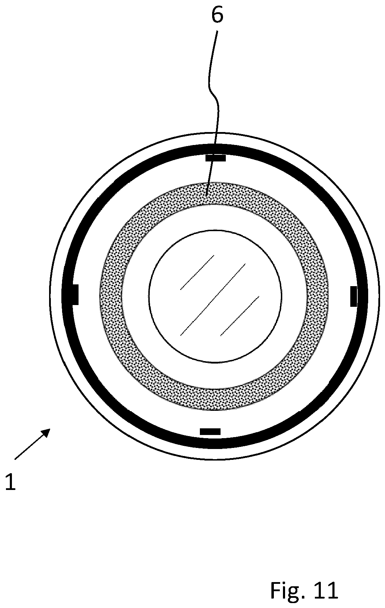

Fig. 11 zeigt in einer schematischen Darstellung eine weiteren Ausführungsform einer selbst akkommodierenden Linse 1.

Der Aktuator 6 ist bei dieser Ausführungsform als ein konzentrischer Ring ausgebildet.

Dieser kann elektroaktive Polymere, wie z.B. Polypyrrol, umfassen, welche aufgrund einer Volumenänderung aufgrund einer elektrochemischen Oxidation und Reduktion durch Anlegen einer Niedrigspannung von weniger als 3 V den Aktuator zusammenziehen, wenn sich das Blickfeld im Nahbereich befindet.

Der Aktuator 6 verläuft ringförmig um ein freies Zentrum und stört daher das Sichtfeld nicht.

Bis auf den Aktuator 6 kann die selbst akkommodierende Linse wie die vorstehend beschriebenen Ausführungsbeispiele ausgebildet sein.

Durch die Erfindung konnte die Implementation der für eine akkommodierende Linse vorhandenden Steuerelemente weiter verbessert werden.

### Bezugszeichenliste

- 1: selbst akkommodierende Linse
- 2: Linsenkörper
- 3: maximale Öffnung Iris
- 4: Steuerungseinrichtung
- 5: Patch-Antenne
- 6: Aktuator
- 7: Mittelpunkt/optische Achse
- 8: Dünnschichtaktuator/Spule
- 9: freier Bereich
- 10: Antenne
- 11: Reflektor
- 12: Patch-Array
- 13: Substrat
- 14: Signalkeule
- 15: biegsames Substrat
- 16: Elektrode
- 17: Hohlraum
- 18: Auge

## Patentansprüche

1. Selbst akkommodierende Linse, insbesondere Kontaktlinse, umfassend einen Linsenkörper, dessen Krümmung in Abhängigkeit zu dem Winkel zu einer benachbarten Linse veränderbar ist, wobei die selbst akkommodierende Linse zur Messung des Winkels zur benachbarten Linse eine Antenne umfasst, welche ein gerichtetes Signal abstrahlt.

2. Selbst akkommodierende Linse nach dem vorstehenden Anspruch, **dadurch gekennzeichnet, dass** die Antenne als Patch-Antenne ausgebildet ist.

3. Selbst akkommodierende Linse nach dem vorstehenden Anspruch, **dadurch gekennzeichnet, dass** die Antenne als Patch-Array, insbesondere als gekrümmtes Patch-Array, ausgebildet ist

4. Selbst akkommodierende Linse, insbesondere nach einem der vorstehenden Ansprüche, umfassend einen Linsenkörper aus einem transparenten elastischen Material, welcher eine Vorderseite und eine Rückseite umfasst, wobei auf der Vorderseite zumindest ein Aktuator zur Veränderung der Krümmung der Linse angeordnet ist.

5. Selbst akkommodierende Linse nach dem vorstehenden Anspruch, **dadurch gekennzeichnet, dass** die Linse eine Mehrzahl von Aktuatoren umfasst,
insbesondere dass sich die Aktuatoren sternförmig über die Vorderseite erstrecken.

6. Selbst akkommodierende Linse nach einem der vorstehenden Ansprüche, **dadurch gekennzeichnet, dass** die Aktuatoren eine Breite von unter 0,5 mm, vorzugsweise von unter 0,3 mm, oder bevorzugt unter 0,1 mm haben.

7. Selbst akkommodierende Linse nach einem der vorstehenden Ansprüche, **dadurch gekennzeichnet, dass** die selbst akkommodierende Linse derart ausgebildet ist, dass die Krümmung schrittweise verstellbar ist, insbesondere in 2 bis 5 Schritten, vorzugsweise in 3 Schritten.

8. Selbst akkommodierende Linse nach einem der vorstehenden Ansprüche, **dadurch gekennzeichnet, dass** der zumindest eine Aktuator als MEMS-Aktuator ausgebildet ist, insbesondere dass der Aktuator als elektrostatischer Aktuator ausgebildet ist.

9. Selbst akkommodierende Linse nach einem der vorstehenden Ansprüche, **dadurch gekennzeichnet, dass** der Aktuator ringförmig ausgebildet ist,
und/oder dass der Aktuator ein elektroaktives Polymer umfasst,

10. Selbst akkommodierende Linse nach einem der vorstehenden Ansprüche, **dadurch gekennzeichnet, dass** als Stell-Eingangsgröße für den Aktuator ein Winkel zu einer anderen Linse verwendet wird,
und/oder dass der Winkel über den Abstand eines Sensors der Linse zu einem anderen Sensor bestimmbar ist und/oder dass die Linse einen in Dünnschichttechnik auf ein Substrat, insbesondere auf ein Glassubstrat, aufgebrachten Energiespeicher umfasst,
und/oder dass der Energiespeicher ringförmig ausgebildet ist.

11. Verfahren zur Steuerung der Brennweite einer selbst akkommodierenden Linse, insbesondere einer selbst akkommodierenden Linse nach einem der vorstehenden Ansprüche, umfassend die Schritte:
- Messen der Signalstärke eines gerichteten Funksignals einer benachbarten Linse,
- Berechnen eines Steuersignals mittels einer Steuereinrichtung,
Ansteuern zumindest eines Aktuators zur Veränderung der Krümmung der Linse.

12. Verfahren nach dem vorstehenden Anspruch, **dadurch gekennzeichnet, dass** mit der Steuereinrichtung der Winkel der optischen Achsen zweier benachbarter Linsen berechnet wird und der zumindest eine Aktuator auf Basis des Winkels angesteuert wird.

13. Verfahren nach einem der vorstehenden Ansprüche, **dadurch gekennzeichnet, dass** die Steuerung stufenweise erfolgt, insbesondere in zumindest 2 Stufen und/oder weniger als 10 Stufen, insbesondere in 2, 3, 4 oder 5 Stufen.

14. Verfahren nach einem der vorstehenden Ansprüche, **dadurch gekennzeichnet, dass** in einer Fernsicht-Stufe der zumindest eine Aktuator in einer Ruheposition ist.

15. Set mit zwei selbst akkommodierenden Linsen, welches zumindest eine selbst akkommodierende Linse nach einem der vorstehenden Ansprüche umfasst.
